# EUROPEAN PATENT SPECIFICATION

(11) **EP 4 766 743 B1**
(45) Date of publication and mention of the grant of the patent: **02.12.2026**
(21) Application number: 25792829.1
(22) Date of filing: 21.10.2025
(51) Int. Cl.: C08B 31/04, A61K 8/73, C08B 37/00, C08L 3/02, C08L 3/06, C08L 5/00, D21H 17/24, D21H 17/28, D21H 17/31, D21H 21/10, D21H 23/04

(54) **PROCESS OF PREPARATION OF A FUNCTIONALIZED POLYMER**
VERFAHREN ZUR HERSTELLUNG EINES FUNKTIONALISIERTEN POLYMERS
PROCÉDÉ DE PRÉPARATION D'UN POLYMÈRE FONCTIONNALISÉ

(30) Priority: 21.10.2024 EP 24306756
(43) Date of publication of application: 01.07.2026
(73) Proprietor: SNF Group, 42160 Andrézieux-Bouthéon (FR)
(72) Inventor: RADENKO, Alexei, 42160 ANDREZIEUX-BOUTHEON (FR); SOUZY, Renaud, 42160 ANDREZIEUX-BOUTHEON (FR); BRAUN, Oliver, 42160 ANDREZIEUX-BOUTHEON (FR)
(74) Representative: Ipsilon
(86) International application number: PCT/EP2025/080286
(87) International publication number: WO 2026/087478

(56) References cited:
- WO-A1-2022/254083

## Description

### Field of the invention

The present invention relates to the use of a functionalized polymer in pulp and paper industry as retention aids.

### Prior art

Polysaccharides are natural polymers that are increasingly being used to replace petrochemical-based polymers. They are abundant resources, and they offer the advantage of being renewable and biodegradable. However, they are generally less efficient and have a shorter shelf life than petrochemical-based polymers.

Numerous developments have been made to overcome this lack of performance, notably through functionalization.

Various techniques such as grafting or chemical functionalization are used to improve polysaccharides performances. Generally, the functionalization is carried out via the hydroxyle functions, present on the polysaccharide skeleton, by reaction with an epoxide bearing a quaternary ammonium group, as described in the document M.-P. Labeau, P. Marion, F. Monnet et al., "Chemicals and Fuels from Bio-Based Building Blocks", Wiley, 2016, pp 615-642. Some production processes also deal with nucleophilic substitution on alkyl chlorides. However, this process generally requires hard conditions, reducing the positive impact of using polysaccharides, in addition due to the hard conditions, during these processes the polysaccharide is partially degraded.

Other milder techniques have been developed, for example document WO22254083 describes the reaction of a polysaccharide with a compound having an electron-withdrawing conjugated group by oxa-Michael addition reaction in the presence of a base. By using this process, the functionalization of the polysaccharide is not well controlled. This leads to functionalized polysaccharides having poor applicative performances and moderate biodegradability.

The applicant has developed a functionalization process that is carried out under mild conditions. This novel approach leads to eco-designed functionalized polymers having good applicative performances while maintaining good biodegradability.

The functionalized polymer exhibits in particular improved performances as retention agent in papermaking.

The invention is in line with the principle of environmental awareness and the impact of industry and mankind on the planet. The functionalized polymer of the invention has improved performances compared to available functionalized polymers, enabling a reduction of polymer quantity used in the application and thus enabling to have a lower environmental impact.

In addition, the present invention is advantageously carried out using materials of biological origin, such as biomass, or recycled materials. The synthesis of the monomers used in the invention are advantageously a biological synthesis, for example, by enzymatic catalysis. The energy used to carry out the process according to the invention is advantageously derived from a heat pump or of renewable origin, for example wind power, photovoltaics, or, in particular for mobile installations, from fuel cell or lithium battery type.

### Disclosure of the invention

The present invention relates to a process for manufacturing a sheet of paper, cardboard or the like, wherein, before the sheet is formed, at least one non-anionic functionalized polymer P3 is added to an aqueous suspension of fibers at one or more injection points, said polymer P3 being obtained by reaction Re2 between
* a polymer P2 having at least one carbon-carbon double bond and
* a compound C2 selected from the group consisting of amino alcohols, amino thiols or amino acids and salts thereof
said polymer P2 being prepared by reaction Re1 between
* a polysaccharide P1 and
* a compound C1 selected from the group consisting of: methacrylic anhydride, itaconic anhydride, maleic anhydride, acryloyl chloride, acryloyl bromide, methacryloyl chloride, methacryloyl bromide, glycidyl (meth)acrylate, and mixtures thereof

### Description of the invention

The non-anionic functionalized polymer P3 is water-soluble or water-swellable. Preferably it is water-soluble.

By "polymer" we mean a homopolymer or a copolymer. A homopolymer is a polymer composed of a single identical repeating unit, while a copolymer is composed of two or more different repeating units.

By "water-soluble polymer", we mean a polymer which gives an aqueous solution without insoluble particles when dissolved with stirring at 25°C at a concentration of 2 g L⁻¹, in deionized water, according to regulations (CE) n° 1907/2006.

By "water-swellable polymer", we mean a polymer that can go through gelatinization when exposed to water and heat. This process leads to the dissolution of the polymer in water, increasing the viscosity of the solution by forming a gel-like medium. This generally occurs by hydrolysis of some intramolecular functions on a non-soluble polymer, allowing water molecules to form hydrogen bonds which solubilizes said polymer. This phenomenon is well known, in particular for starch. Water-swellable according to the invention does not refer to superabsorbent polymers which form a three-dimensional network of covalent bonds trapping water molecule and forming a solid. Superabsorbent/hydrogel polymers are therefore excluded from the scope of the invention.

By "esterification or thio-esterification reaction", we mean any chemical reaction where at least one of the products formed is an ester or a thio ester.

By "amidification reaction", we designate any chemical reaction where at least one of the products formed is an amide.

By "Michael addition" reaction, we designate a conjugate nucleophilic addition reaction involving a nucleophile (Michael donors) and a Michael acceptor, oxa-Michael addition, aza-Michael addition and thia-Michael addition are included.

By "X and/or Y" we mean "X," or "Y," or "X and Y."

The invention also covers all possible combinations of the other than disclosed embodiments, whether they are preferred embodiments or given by way of example. Furthermore, when ranges of values are indicated, the limits are part of these ranges. The disclosure also includes all combinations between the limits of these value ranges. For example, the value ranges "1-20, preferably 5-15", imply disclosure of the ranges "1-5", "1-15", "5-20", and "15-20" and the values 1, 5, 15 and 20.

All the particular and/or preferred modes described in the invention are combinable, provided they are not incompatible.

### Process of preparation of the non-anionic functionalized polymer P3

The present invention relates to a process for manufacturing a sheet of paper, cardboard or the like, wherein, before the sheet is formed, at least one non-anionic functionalized polymer P3 is added to an aqueous suspension of fibers at one or more injection points, said polymer P3 being obtained by reaction Re2 between
* a polymer P2 having at least one carbon-carbon double bond and
* a compound C2 selected from the group consisting of amino alcohols, amino thiols or amino acids and salts thereof
said polymer P2 being prepared by reaction Re1 between
* a polysaccharide P1 and
* a compound C1 selected from the group consisting of: methacrylic anhydride, itaconic anhydride, maleic anhydride, acryloyl chloride, acryloyl bromide, methacryloyl chloride, methacryloyl bromide, glycidyl (meth)acrylate, and mixtures thereof.

By "non-anionic functionalized polymer P3", we mean that the polymer P3 can be cationic, amphoteric, non-ionic. The polymer P3 can comprise anionic functions but cannot contain only anionic functions nor a mixture of only non-ionic and anionic functions, which are thereof excluded from the scope of the invention.

### Preparation of polymer P2

The polymer P2 having at least one carbon-carbon double bond is prepared by reaction Re1 between a polysaccharide P1 and a compound C1 selected from the group consisting of: methacrylic anhydride, itaconic anhydride, maleic anhydride, acryloyl chloride, acryloyl bromide, methacryloyl chloride, methacryloyl bromide, glycidyl (meth)acrylate, and mixtures thereof.

The reaction is advantageously a condensation reaction, preferably chosen from esterification, thio-esterification or amide formation reactions.

The reaction is advantageously carried out in a polar solvent PS1 chosen from: water, isopropanol, tert-butanol, ethanol, acetone, dimethyl sulfoxide, tetrahydrofuran, dimethylformamide and mixture thereof. Preferably it is water.

The reaction is advantageously carried out at a temperature comprised between -10 and 100°C, preferably between 10 and 50°C, more preferably between 15 and 40°C.

The reaction temperature between the polysaccharide P1 and the compound C1 will be adjusted by the skilled man of the art in order to have a polar solvent PS1 in liquid form.

The pH of the reaction is advantageously controlled between 6 and 12, preferably between 7 and 10, more preferably between 7 and 9.

The pH can be adjusted using a base for example an alkali hydroxide or an alkaline earth metal hydroxide or amine base, like sodium hydroxide or potassium hydroxide, sodium methoxide, 1,4-diazabicyclo[2.2.2]octane (DABCO), triethylamine (TEA), sodium carbonate (Na₂CO₃), pyridine (C₅H₅N), sodium bicarbonate (NaHCO₃), potassium carbonate (K₂CO₃), potassium bicarbonate (KHCO₃), sodium ethoxide, and. potassium tert-butoxide (tBuOK), diazabicyclo undecene (DBU), sodium methoxide or a mixture thereof. Preferably, the base is sodium hydroxide.

The reaction lasts advantageously between 1 minute and 1500 minutes, preferably between 30 minutes and 240 minutes, more preferably between 60 minutes and 120 minutes.

The reaction is advantageously carried out at atmospheric pressure.

The polysaccharide P1 can be water-soluble or water-swellable. Preferably it is water-soluble.

By "polysaccharide" we mean a polymer comprising monosaccharide repeating units linked via glycosidic bonds. Polysaccharides of the invention contains at least 4 monosaccharides units.

Advantageously, the polysaccharide contains at least one functional group chosen from hydroxyl -OH, thiol -SH and amine -NH_{2.}

The polysaccharide may be a modified polysaccharide.

By "modified polysaccharide" we mean a polysaccharide that has undergone one or more treatments, which may be physical and/or chemical and/or enzymatic.

The polysaccharide can be extracted from plants, animals or produced by micro-organisms such as bacteria, fungi, prokaryotes and eukaryotes. For example, xanthan gum can be produced by Xanthomonas campestris, gellan by Sphingomonas paucimobllis, xyloglucan can be extracted from tamarind seeds.

Preferably, the polysaccharide is selected from the group consisting of: starch, starch derivatives, maltodextrin, xanthan gum, dextran, galactomannan, glucomannan, alginates, chitosan, cellulose, cellulose derivatives such as carboxy methyl cellulose, and mixtures thereof.

According to a preferred embodiment, the polysaccharide can undergo chemical grafting reactions with various synthetic substituents, for example, carboxyalkyl starch, like carboxymethyl starch, carboxyalkyl cellulose, like carboxymethyl cellulose, carboxyalkyl guar, like carboxymethyl guar, hydroxypropyl methylcellulose, carboxyalkyl hydroxyalkyl cellulose, like carboxymethyl hydroxypropyl cellulose, carboxyalkyl hydroxyalkyl guars, like carboxymethyl hydroxypropyl guar. This type of reaction is familiar to those skilled in the art as classic chemical reactions applied to natural polymers.

In general, the number average molecular weight of the polysaccharide P1 ranges from 500 to 30 000 000 g/mol.

In a preferred embodiment, the polysaccharide P1 has a number average molecular weight (Mₙ) (determined by gel permeation chromatography) comprised between 5 000 and 30 000 000 g/mol, preferably between 10 000 and 10 000 000 g/mol, more preferably between 100 000 and 1 000 000 g/mol.

The analytical conditions are as follows:
- 1 Shodex pre-column referenced SB807-G
- 2 Shodex OHpak columns mounted in series, referenced SB-807 HQ / SB-805 HQ)
- The columns are coupled with a refractive index detector referenced Optilab T-rEX and Dawn Heleos II 18 angles marketed by the company Wyatt Technology.

The molar ratio between the compound C1 and a monosaccharide unit of the polysaccharide P1 is advantageously comprised between 2:1 and 1:10000, preferably between 1:1 and 1:1000, more preferably between 1:3 and 1:100.

When the compound C1 is hydrophobic, the molar ratio between polysaccharide P1 and compound C1 is adjusted in order to obtain a polymer P2 which is water-soluble.

The degree of substitution (DS) of the polysaccharide P1 by the compound C1 is advantageously comprised between 0.00001 and 1, preferably between 0.0001 and 2, more preferably between 0.0001 and 1, more preferably between 0.01 and 0.33, more preferably between 0.05 and 1, even more preferably between 0.1 and 0.5.

By "degree of substitution" we mean the average amount of functional groups (hydroxyl, thiol and amine) on the polysaccharide P1 that reacted with the compound C1 compared to the total number of unreacted functional groups on the polysaccharide P1 before the reaction. The maximum value is therefore 1. In this case, all the functional groups on the polysaccharide P1 have reacted with compound C1.

The degree of substitution is determined by 1H NMR spectroscopy (Bruker 400MHz, equipped with multinuclear probe).

The reaction of polysaccharide P1 with compound C1 gives a polymer P2 functionalized with compound C1.

### Reaction between the polymer P2 and the compound C2 to form the non-anionic functionalized polymer P3

Compound C2 is selected from the group consisting of amino alcohols, amino thiols or amino acids and salts thereof
The compound C2 can have an anionic, a non-ionic, or cationic charge. Preferably, in conditions of use, C2 has cationic units.

Preferably, the cationic charge is borne by the nitrogen of the amine group, that is the amine is in the form of ammonium (quaternary ammonium).

The compound C2 can comprise more than one hydroxy group and/or thiol group and/or amino group.

Preferably, compound C2 is an amine thiol wherein the amine is neutral or in the form of ammonium, preferably in the form of ammonium.

In some embodiments, the compound C2 is an amino acid containing at least one thiol group.

The compound C2 advantageously comprises at most 3 thiol groups, preferably at most 2 thiol groups, more preferably 1 thiol group.

When the compound C2 comprises more than 1 thiol group, its quantity is adjusted in order to prevent the formation of a three-dimensional network in order to obtain a functionalized polymer which is water-soluble or water-swellable and not a three-dimensional network of covalent bonds.

In a preferred embodiment, the compound C2 is chosen from:

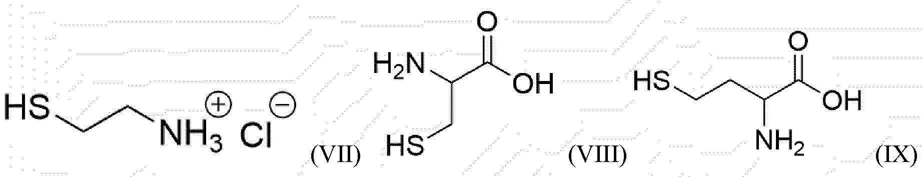

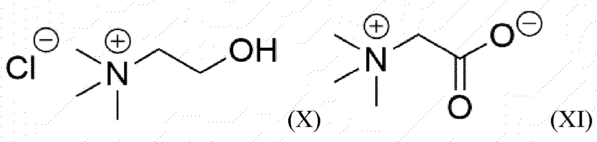

The reaction between the polymer P2 and the compound C2 is a Michael addition reaction, wherein the polymer P2 bearing at least one carbon-carbon double bond is the Michael acceptor and the compound C2 plays the role of Michael donor. They react to form the non-anionic functionalized polymer P3 by a nucleophilic addition of the Michael donor on the Michael acceptor to form a Michael adduct corresponding to the non-anionic functionalized polymer P3.

The molar ratio between the compound C1 of polymer P2 and the compound C2 is advantageously comprised between 1:2 and 10: 1, preferably between 1:1 and 2:1. The molar ratio is preferably 1:1.

The degree of substitution of (DS) of the polymer P2 by the compound C2 is the same as the degree of substitution of the polysaccharide P1 by the compound C1.

When the compound C2 is hydrophobic, the molar ratio between the polymer P2 and the compound C2 is adjusted in order to obtain a non-anionic functionalized polymer P3 which is water-soluble or water-swellable.

The reaction between the polymer P2 and the compound C2 is advantageously carried out in a polar solvent PS2 chosen from: water, isopropanol, acetone, dimethyl sulfoxide, tetrahydrofuran, dimethylformamide and mixture thereof. Preferably it is water.

The reaction between the polymer P2 and the compound C2 is advantageously carried out at a temperature comprised between -5 and 100°C, preferably between 10 and 60°C, more preferably between 15 and 30°C.

The reaction temperature during the reaction between the polymer P2 and the compound C2 will be adjusted by the skilled man of the art in order to have a polar solvent PS2 in liquid form.

The reaction between the polymer P2 and the compound C2 is advantageously adjusted to a pH comprised between 6 and 12, preferably between 6.5 and 10, more preferably between 7 and 9.

The reaction between the polymer P2 and the compound C2 lasts advantageously between 10 minutes and 3 000 minutes, preferably between 60 minutes and 600 minutes, more preferably between 60 minutes and 400 minutes.

The reaction between the polymer P2 and the compound C2 is advantageously carried out at atmospheric pressure.

The non-anionic functionalized water-polymer P3 can be a non-ionic, a cationic, an amphoteric or a zwitterionic polymer depending on the choice of compound C1 and C2, and/or subsequent optionally quaternarization or hydrolysis reaction on the non-anionic functionalized polymer P3.

In a particular embodiment, the non-anionic functionalized polymer P3 is quaternized.

Those skilled in the art will know how to prepare quaternized functionalized polymer, for example using a quaternizing agent of the R-X type, where R is an alkyl group and X is a halogen or sulfate.

The term "quaternizing agent" refers to a molecule capable of alkylating a tertiary amine.

The quaternizing agent may be chosen from dialkyl sulfates containing from 1 to 6 carbon atoms or alkyl halides containing from 1 to 6 carbon atoms. Preferably, the quaternizing agent is chosen from methyl chloride, benzyl chloride, dimethyl sulfate or diethyl sulfate.

In a particular embodiment the non-anionic functionalized polymer P3 is washed to remove undesired salts, side products and unreacted reagents.

In a preferred embodiment, compound C1 and compound C2 are chosen from at least partially renewable and non-fossil sources.

In the context of the invention, the terms "renewable and non-fossil" are used to designate the origin of a chemical compound derived from biomass or from synthesis gas (syngas), i.e. resulting from one or more chemical transformations carried out on one or more natural and non-fossil raw materials. The terms "bio-sourced" or "bio-resourced" can also be used to characterize the renewable and non-fossil origin of a chemical compound. The renewable and non-fossil origin of a compound includes renewable and non-fossil raw materials stemming from the circular economy, which have been previously recycled, once or several times, in a biomass material recycling process, such as materials from polymer depolymerization or pyrolysis oil processing.

According to the invention, the "at least partially renewable and non-fossil" quality of a compound means a bio-sourced carbon content preferably between 5wt% and 100wt% relative to the total carbon weight of said compound.

In the context of the invention, the ASTM D6866-21 standard Method B is used to characterize the bio-sourced nature of a chemical compound and to determine the bio-sourced carbon content of said compound. The value is expressed as a weight percentage (wt%) of bio-sourced carbon relative to the total carbon weight in said compound.

In a particularly preferred embodiment, compound C1 and compound C2 are 100 wt% renewable and non-fossil source.

The number average molecular weight (Mn) of the polymer P3 depends on the nature of the polysaccharide P1 used and the degree of substitution.

In some preferred embodiments, the polymer P3 has a number average molecular weight (Mₙ) (determined by gel permeation chromatography) comprised between 5 500 and 30 000 000 g/mol, preferably between 10 000 and 10 000 000 g/mol, more preferably between 105 000 and 1 000 000 g/mol.

The analytical conditions are as follows:
- 1 Shodex pre-column referenced SB807-G
- 2 Shodex OHpak columns mounted in series, referenced SB-807 HQ / SB-805 HQ)
- The columns are coupled with a refractive index detector referenced Optilab T-rEX and Dawn Heleos II 18 angles marketed by the company Wyatt Technology.

### Process for manufacturing a sheet of paper

The invention relates to a process for manufacturing a sheet of paper, cardboard or the like, according to which, before a sheet is formed, at least one non-anionic functionalized polymer P3, obtained as described above, is added to a fiber suspension at one or more injection points.

The process of manufacturing a sheet of paper, cardboard or the like comprises advantageously the following successive steps:
(1) adding a non-anionic functionalized polymer P3, to an aqueous suspension of cellulosic fibers;
(2) forming a sheet of paper, cardboard or the like;
(3) drying said sheet of paper, cardboard or the like.

The different steps in a process of manufacturing paper, cardboard or the like are known and comply with techniques that are part of the knowledge of the skilled person in the art, it is not necessary to describe them in further detail, because they remain known and classic of what the person skilled in the art knows. If necessary, they can refer to the document: Handbook for Pulp & Paper Technologists, 4th Edition, G.A. Smook.

The quantity of polymer P3 added in step (1) is preferably between 0.1 kg/t and 50 kg/t, preferably between 0.5 kg/t and 30 kg/t, more preferably between 1 kg/t and 15 kg/t.

By "kg/t" is meant kilogram of polymer P3 per ton (1 000 kg) of dry cellulosic material.

The addition of the non-anionic functionalized polymer P3 to the aqueous suspension of cellulosic fibers can be done in different ways and in particular according to the typical methods known to the person skilled in the art.

The non-anionic functionalized polymer P3 can be added to the cellulose material in the form of a diluted or undiluted aqueous solution. It can be applied by an impregnation technic, or it can be added directly to the fibrous suspension at any point of the process of producing paper where dry strength agents are usually introduced.

Thus, the non-anionic functionalized polymer P3 can be introduced into the thick stock or into the thin stock. It can be added at the mixing pump, before the headbox or filter sieve. Preferably, the non-anionic functionalized polymer P3 is introduced before the headbox.

Preferably, the non-anionic functionalized polymer P3 is industrially injected into the fibrous suspension, i.e., before its dilution by the pulp water (thick stock). The consistency of the pulp is on the order of 1 to 5% by weight of the cellulose fibers.

The papermaking method can be implemented with any type of paper pulp, such as, for example, virgin fiber pulp (Kraft, Bisulfite), recycled fibers, de-inked pulps, mechanical and thermo-mechanical pulps.

The non-anionic functionalized polymer P3 is added before the formation of the sheet, directly into the fibrous suspension.

It can be added at a single injection point, or at two injection points. The papermaking method according to the invention may also include the addition of other additives and/or polymers, according to needs; by way of example and without limitation, we can mention: biocides, coagulants, other retention agents, flocculants, starch, dry strength agents, wet strength agents, dispersants, defoamers, fixatives, fillers or dyes.

### Others

In some embodiments, the non-anionic functionalized polymer P3, obtained as described herein, may be used in: drilling wells; cementing wells; oil and/or gas production (enhanced oil recovery, conformance, diversion, hydraulic fracturing); water treatment; treatment of fermentation broth; sludge treatment; construction; paper or cardboard manufacture; batteries; wood treatment; treatment of hydraulic compositions (concrete, cement, mortar and aggregates); mining; formulation of cosmetic products; formulation of detergents; textile manufacture; geothermal energy; sanitary napkin manufacturing or in agriculture.

In some embodiments, the non-anionic functionalized polymer P3, obtained as described herein, may be used as a flocculant, coagulant, binding agent, draining agent, viscosityreducing agent, friction-reducing agent, absorbent agent, filler retention agent, conditioning agent, stabilising agent, stabilising agent, film-forming agent, sizing agent, (super)plasticizing agent, dehydration agent, clay inhibitor, thickening agent, or dispersant.

In some embodiments, the non-anionic functionalized polymer P3 may be used in a process for treating a suspension of solid particles in water or wastewater, comprising contacting said suspension with at least one non-anionic functionalized polymer P3 obtained as described herein.

In some embodiments, the non-anionic functionalized polymer P3 may be used in a home care and personal care composition.

In some embodiments, the non-anionic functionalized polymer P3 may also be used in a method of conditioning fibers, said method comprising contacting said fibers with polymer P3 obtained as described herein, the fibers being advantageously hairs.

### Examples

The following examples illustrate the invention without limiting its scope.

### Starting materials

- Starch were provided by:
   * Starch-1: Roquette Frères, commercial name Stabilys^{®} A053;
   * Starch-2: Roquette Frères, commercial name Stabilys^{®} A020.
- Xanthan gums were provided by:
   * Xanthan gum-1: Jungbunzlauer, commercial name Xanthan Gum FFPC;
   * Xanthan gum-2: CP Kelco, commercial name KELZAN^{®} AP-AS.
- Maltodexrins were provided by:
   * Maltodextrin-1: Tate & Lyle, commercial name Maltosweet^{®} 180;
   * Maltodextrin-2: Prodechim, commercial name MALTODEXTRINE 18DE;
   * Maltodextrin-3: Cargill, commercial name C-Dry^{™} MD 01958.
- Methacrylic anhydride was provided by Evonik Operations GmbH, commercial name VISIOMER^{®} MAAH;
- Itaconic anhydride was provided by TCI.
- Maleic anhydride was provided by Sigma-Aldrich.
- Cysteamine was provided by ABCR in the form of its hydrochloride salt.
- L-Cysteine was provided by Sigma-Aldrich
- Polyquaternium 10 (PQ10)

### Analysis

Final and intermediate products were analyzed by 1H NMR spectroscopy using Bruker 400MHz spectrometer equipped with multinuclear probe.

### Procedure for the preparation of functionalized polymers P3-1 to P3-20

All reactions were conducted at room temperature (19 - 23°C) without heating or cooling.

### Process of preparation of non-anionic functionalized polymer P3-1

### Preparation of polymer P2

210 g of water was introduced into a 1 L reactor equipped with a mechanical stirrer and a pH-meter.

210 g of Starch-1 **(P1)** was added slowly and mixed at 500 rpm throughout the reaction.

The pH was adjusted to 8 with a 50% NaOH aqueous solution.

The pH was maintained between 7 and 9 during the reaction.
2 g of methacrylic anhydride (compound **C**1) were added dropwise during 1 h. The molar ratio between a monosaccharide unit and the compound **C1** was 1:1.3.

At the end of the reaction, the polymer **P2** was obtained.

The degree of substitution (DS) was measured by ¹H NMR to be 0.003.

### Preparation process for the synthesis of non-anionic functionalized polymer P3-1

1.92 g of cysteamine hydrochloride (compound **C2**) was added to the polymer **P2.** The reaction mixture was stirred for 2 h to give the non-anionic functionalized polymer **P3-1.**

Functionalized polymers **P3-2** to **P3-20** were synthetized according to the process described for polymer **P3-1.**

Their composition and nature are presented in Table 1.

Polymers obtained with the functionalization process according to the invention are compared to polyquaternium-10 **(PQ10),** a quaternized hydroxyethyl cellulose. **PQ10** is used as reference as this compound represents the most and best functionalized biopolymer available on the market.

**Table 1: Composition and nature of functionalized polymers P3-1 to P3-20**

| **Functionalized polymers P3** | **P1** | **C1** | **DS of P2** | **C2** | **Molar ratio monosaccharide:C1** | **Mn of P3** |
|---|---|---|---|---|---|---|
| P3-1 | Starch-1 | Methacrylic anhydride | 0.003 | Cysteamine | 1:1.3 | 15 000 |
| P3-2 | Starch-1 | Methacrylic anhydride | 0.033 | Cysteamine | 1:1.3 | 15 000 |
| P3-3 | Starch-1 | Methacrylic anhydride | 0.167 | Cysteamine | 1:1.3 | 15 000 |
| P3-4 | Starch-1 | Methacrylic anhydride | 0.333 | Cysteamine | 1:1.3 | 15 000 |
| P3-5 | Starch-1 | Maleic anhydride | 0.167 | Cysteamine | 1:1.3 | 15 000 |
| P3-6 | Starch-1 | Itaconic anhydride | 0.167 | Cysteamine | 1:1.3 | 15 000 |
| P3-7 | Starch-2 | Maleic anhydride | 0.167 | Cysteamine | 1:1.3 | 400 000 |
| P3-8 | Starch-2 | Methacrylic anhydride | 0.167 | Cysteamine | 1:1.3 | 400 000 |
| P3-9 | Maltodextrin-1 | Methacrylic anhydride | 0.033 | Cysteamine | 1:1.3 | 1 300 |
| P3-10 | Maltodextrin-1 | Methacrylic anhydride | 0.167 | Cysteamine | 1:1.3 | 1 300 |
| P3-11 | Maltodextrin-2 | Methacrylic anhydride | 0.167 | Cysteamine | 1:1.3 | 1 300 |
| P3-12 | Maltodextrin-3 | Methacrylic anhydride | 0.167 | Cysteamine | 1:1.3 | 2 600 |
| P3-13 | Maltodextrin-1 | Maleic anhydride | 0.033 | Cysteamine | 1:1.3 | 1 300 |
| P3-14 | Xanthan gum-2 | Methacrylic anhydride | 0.033 | Cysteamine | 1:1.3 | 3 000 000 |
| P3-15 | Xanthan gum-1 | Methacrylic anhydride | 0.033 | Cysteamine | 1:1.3 | 3 500 000 |
| P3-16 | Xanthan gum-2 | Methacrylic anhydride | 0.167 | Cysteamine | 1:1.3 | 3 000 000 |
| P3-17 | Xanthan gum-2 | Itaconic anhydride | 0.033 | Cysteamine | 1:1.3 | 3 000 000 |
| P3-18 | Starch-1 | Methacrylic anhydride | 0.003 | Cysteine | 1:1.3 | 150 000 |
| P3-19 | Starch-1 | Methacrylic anhydride | 0.033 | Cysteine | 1:1.3 | 150 000 |
| P3-20 | Starch-1 | Methacrylic anhydride | 0.167 | Cysteine | 1:1.3 | 150 000 |

### Example 1 - Biodegradation tests

The biodegradability of the polymers was evaluated according to OECD 301F procedure. The results are presented as the oxygen consumption relative to the theoretical oxygen demand (ThOD).

A polymer is considered as inherently biodegradable if the biodegradability reaches between 20 and 60% in 28 days according to OECD 301F.

A polymer is considered as readily biodegradable if the biodegradability reaches at least 60% in 28 days according to OECD 301F.

A polymer is considered as ultimately biodegradable if the biodegradability reaches at least 60% in 90 days according to OECD 301.

The results are summarized in Table 2.

**Table 2: Biodegradability of polymers P3-1 to P3-20**

| **Functionalized polymers** | **Biodegradability test at 28 days (%)** | **Biodegradability test at 60 days (%)** |
|---|---|---|
| P3-1 | 88.5 | 91.6 |
| P3-2 | 73.4 | 76.6 |
| P3-3 | 54.7 | 67.7 |
| P3-4 | 47.8 | 62.9 |
| P3-5 | 51.7 | 65.4 |
| P3-6 | 53.2 | 69.5 |
| P3-7 | 51.6 | 61.5 |
| P3-8 | 54.1 | 70.9 |
| P3-9 | 77.3 | 81.3 |
| P3-10 | 62.6 | 72.2 |
| P3-11 | 61.3 | 71.6 |
| P3-12 | 63.7 | 70.4 |
| P3-13 | 69.7 | 74.2 |
| P3-14 | 42.3 | 92.3 |
| P3-15 | 46.7 | 61.5 |
| P3-16 | 42.2 | 63.3 |
| P3-17 | 47.3 | 63.5 |
| P3-18 | 90.3 | 91.9 |
| P3-19 | 85.5 | 89.3 |
| P3-20 | 60.6 | 68.8 |

All the polymers synthesized according to the process of the invention show a good biodegradability at 28 days and are almost completely biodegraded after 60 days.

### Application tests

### Example 2 - Paper industry

The properties of Filler Retention (FR) of the functionalized polymers of the invention were evaluated. The higher the values, the better the performance. For the test procedure for evaluating the filler retention, the various results were obtained using a Britt Jar, with a stirring rate of 1000 rpm.

The % FR is calculated by the following formula: 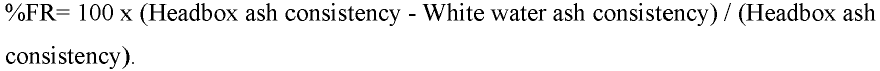 %FR= 100 x (Headbox ash consistency - White water ash consistency) / (Headbox ash consistency).

The polymers' performances are compared to a blank.

The results are presented in Table 3.

**Table 3: Comparison of the Filler Retention results between the polymers P3-3, P3-5, P3-8, P3-12, P3-16 and P3-20 of the invention and PQ10.**

| **Trial** | **Polymer** | **FR (%)** |
|---|---|---|
| 26 | P3-3 | 24.9 |
| 27 | P3-5 | 22.2 |
| 28 | P3-8 | 24.7 |
| 29 | P3-12 | 23.9 |
| 30 | P3-16 | 25.2 |
| 31 | P3-20 | 24.1 |
| 32 | PQ10 | 16.3 |

The results demonstrate that the biopolymers of the invention display good performances in filler retention compared to **PQ10.**

## Claims

1. Process for manufacturing a sheet of paper or cardboard, wherein, before the sheet is formed, at least one non-anionic functionalized polymer P3 is added to an aqueous suspension of fibers at one or more injection points,
said polymer P3 being obtained by reaction Re2 between
* a polymer P2 having at least one carbon-carbon double bond and
* a compound C2 selected from the group consisting of amino alcohols, amino thiols or amino acids and salts thereof
said polymer P2 being prepared by reaction Re1 between
* a polysaccharide P1 and
* a compound C1 selected from the group consisting of: methacrylic anhydride, itaconic anhydride, maleic anhydride, acryloyl chloride, acryloyl bromide, methacryloyl chloride, methacryloyl bromide, glycidyl (meth)acrylate, and mixtures thereof.

2. Process according to claim 1, ***wherein*** the polysaccharide P1 contains at least one functional group chosen from hydroxyl -OH, thiol -SH and amine -NH₂.

3. Process according to claim 1 or claim 2, ***wherein*** the polysaccharide P1 is selected from the group consisting of: starch, starch derivatives, maltodextrin, xanthan gum, dextran, galactomannan, glucomannan, alginates, chitosan cellulose, cellulose derivatives, and mixtures thereof.

4. Process according to any one of the preceding claims, ***wherein*** the polysaccharide P1 has a number average molecular weight Mₙ comprised between 10 000 and 10 000 000 g/mol determined by gel permeation chromatography.

5. Process according to claim 1 or 2, ***wherein,*** in reaction Re1, compound C1 and a monosaccharide unit of the polysaccharide P1 have a molar ratio of between 2:1 and 1:10000.

6. Process according to any one of the preceding claims, ***wherein*** the polymer P2 has a degree of substitution by the compound C1 of between 0.0001 and 1.

7. Process according to any one of the preceding claims, ***wherein*** the compound C2 has a cationic charge.

8. Process according to any one of the preceding claims, ***wherein*** the compound C2 comprises at most one thiol group.

9. Process according to any one of the preceding claims, ***wherein*** the compound C2 is selected from the group consisting of:

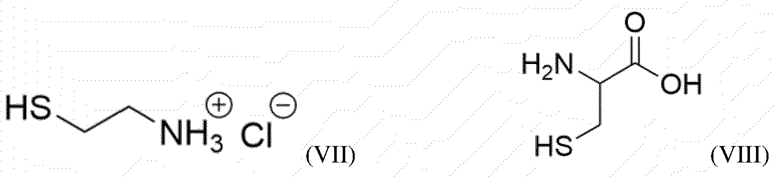

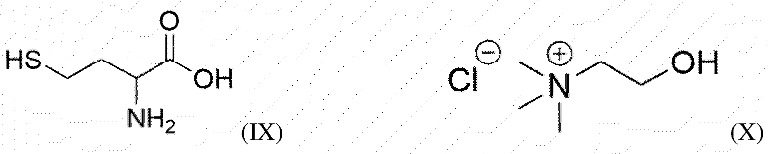

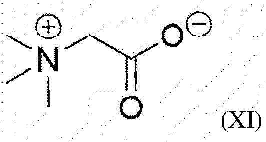

10. Process according to any one of the preceding claims, ***wherein*** compound C1 and compound C2 are compounds at least partially of renewable and of non-fossil sources.

11. Process according to any one of the preceding claims, ***wherein*** compound C1 and compound C2 have a carbon content from renewable and non-fossil origin of between 5% by weight and 100% by weight according to ASTM D6866-21, method B.

12. Process according to any one of the preceding claims, ***wherein*** the process comprises the following successive steps:
(1) adding a non-anionic functionalized polymer P3, to an aqueous suspension of cellulosic fibers and;
(2) forming a sheet of paper, cardboard or the like;
(3) drying said sheet of paper, cardboard or the like.

13. Process according to any one of the preceding claims, ***wherein*** the process is performed using energy derived from a heat pump, a waste heat network, a renewable source, a fuel cell, a lithium battery or a nuclear power plant.

## Patentansprüche

1. Verfahren zur Herstellung einer Papier- oder Kartonbahn, bei dem vor der Bahnbildung mindestens ein nicht-anionisch funktionalisiertes Polymer P3 an einer oder mehreren Einspritzstellen einer wässrigen Fasersuspension zugesetzt wird,
wobei das Polymer P3 durch Reaktion Re2 zwischen
* einem Polymer P2 mit mindestens einer Kohlenstoff-Kohlenstoff-Doppelbindung und
* einer Verbindung C2 aus der Gruppe bestehend aus Aminoalkoholen, Aminothiolen oder Aminosäuren und deren Salzen,
gewonnen wird,
wobei das Polymer P2 durch Reaktion Re1 zwischen
* einem Polysaccharid P1 und
* einer Verbindung C1 aus der Gruppe bestehend aus: Methacrylsäureanhydrid, Itaconsäureanhydrid, Maleinsäureanhydrid, Acryloylchlorid, Acryloylbromid, Methacryloylchlorid, Methacryloylbromid, Glycidyl(meth)acrylat und deren Mischungen,
hergestellt wird.

2. Verfahren nach Anspruch 1, ***wobei*** das Polysaccharid P1 mindestens eine Funktionsgruppe enthält, die aus Hydroxyl (-OH), Thiol (-SH) und Amin (-NH₂) ausgewählt ist.

3. Verfahren nach Anspruch 1 oder Anspruch 2, ***wobei*** das Polysaccharid P1 aus der Gruppe ausgewählt ist, die aus Stärke, Stärkederivaten, Maltodextrin, Xanthangummi, Dextran, Galactomannan, Glucomannan, Alginaten, Chitosan-Cellulose, Cellulosederivaten und deren Mischungen besteht.

4. Verfahren nach einem der vorstehenden Ansprüche, ***wobei*** das Polysaccharid P1 ein durchschnittliches Molekulargewicht Mn zwischen 10.000 und 10.000.000 g/mol aufweist, das durch Gelpermeationschromatographie bestimmt wird.

5. Verfahren nach Anspruch 1 oder 2, ***wobei*** die Verbindung C1 und eine Monosaccharideinheit des Polysaccharids P1 in der Reaktion Re1 ein Molverhältnis zwischen 2:1 und 1:10.000 aufweisen.

6. Verfahren nach einem der vorstehenden Ansprüche, ***wobei*** der Substitutionsgrad des Polymers P2 durch die Verbindung C1 zwischen 0,0001 und 1 liegt.

7. Verfahren nach einem der vorstehenden Ansprüche, ***wobei*** die Verbindung C2 eine kationische Ladung aufweist.

8. Verfahren nach einem der vorstehenden Ansprüche, ***wobei*** die Verbindung C2 höchstens eine Thiolgruppe enthält.

9. Verfahren nach einem der vorstehenden Ansprüche, ***wobei*** die Verbindung C2 aus folgender Gruppe ausgewählt ist:

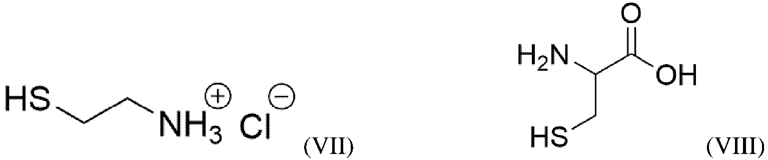

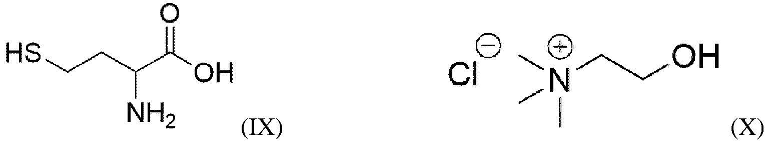

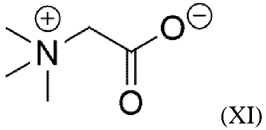

10. Verfahren nach einem der vorstehenden Ansprüche, ***wobei*** die Verbindung C1 und die Verbindung C2 Verbindungen sind, die zumindest teilweise aus erneuerbaren und nicht-fossilen Quellen stammen.

11. Verfahren nach einem der vorstehenden Ansprüche, ***wobei*** die Verbindung C1 und die Verbindung C2 einen Kohlenstoffgehalt aus erneuerbaren und nicht-fossilen Quellen zwischen 5 Gew.-% und 100 Gew.-% nach ASTM D6866-21, Verfahren B, aufweisen.

12. Verfahren nach einem der vorstehenden Ansprüche, ***wobei*** das Verfahren folgende aufeinanderfolgende Schritte umfasst:
(1) Zugabe eines nicht-anionisch funktionalisierten Polymers P3 zu einer wässrigen Suspension aus Zellulosefasern und;
(2) Herstellen einer Papier- oder Kartonbahn oder Ähnlichem;
(3) Trocknen der Papierbahn, Kartonbahn oder Ähnlichem.

13. Verfahren nach einem der vorstehenden Ansprüche, ***wobei*** das Verfahren mit Energie durchgeführt wird, die aus einer Wärmepumpe, einem Abwärmenetz, einer erneuerbaren Energiequelle, einer Brennstoffzelle, einer Lithiumbatterie oder einem Kernkraftwerk gewonnen wird.

## Revendications

1. Procédé de fabrication d'une feuille de papier ou de carton, dans lequel, avant la formation de la feuille, au moins un polymère fonctionnalisé non anionique P3 est ajouté à une suspension aqueuse de fibres en un ou plusieurs points d'injection,
ledit polymère P3 étant obtenu par réaction Re2 entre
* un polymère P2 présentant au moins une double liaison carbone-carbone et
* un composé C2 choisi dans le groupe constitué par les aminoalcools, les aminothiols ou les acides aminés et leurs sels,
ledit polymère P2 étant préparé par réaction Re1 entre
* un polysaccharide P1 et
* un composé C1 choisi dans le groupe constitué par : l'anhydride méthacrylique, l'anhydride itaconique, l'anhydride maléique, le chlorure d'acryloyle, le bromure d'acryloyle, le chlorure de méthacryloyle, le bromure de méthacryloyle, le (méth)acrylate de glycidyle, et leurs mélanges.

2. Procédé selon la revendication 1, ***dans lequel*** le polysaccharide P1 contient au moins un groupe fonctionnel choisi parmi les groupes hydroxyle -OH, thiol -SH et amine -NH₂.

3. Procédé selon la revendication 1 ou la revendication 2, ***dans lequel*** le polysaccharide P1 est choisi dans le groupe constitué par : l'amidon, les dérivés d'amidon, la maltodextrine, la gomme xanthane, le dextrane, le galactomannane, le glucomannane, les alginates, le chitosane la cellulose, les dérivés de cellulose, et leurs mélanges.

4. Procédé selon l'une quelconque des revendications précédentes, ***dans lequel*** le polysaccharide P1 présente une masse moléculaire moyenne en nombre Mₙ comprise entre 10 000 et 10 000 000 g/mol, déterminée par chromatographie par perméation de gel.

5. Procédé selon la revendication 1 ou 2, ***dans lequel***, dans la réaction Re1, le composé C1 et une unité monosaccharidique du polysaccharide P1 sont dans un rapport molaire compris entre 2:1 et 1:10 000.

6. Procédé selon l'une quelconque des revendications précédentes, ***dans lequel*** le polymère P2 présente un degré de substitution par le composé C1 compris entre 0,0001 et 1.

7. Procédé selon l'une quelconque des revendications précédentes, ***dans lequel*** le composé C2 porte une charge cationique.

8. Procédé selon l'une quelconque des revendications précédentes, ***dans lequel*** le composé C2 comprend au plus un groupe thiol.

9. Procédé selon l'une quelconque des revendications précédentes, ***dans lequel*** le composé C2 est choisi dans le groupe constitué par :

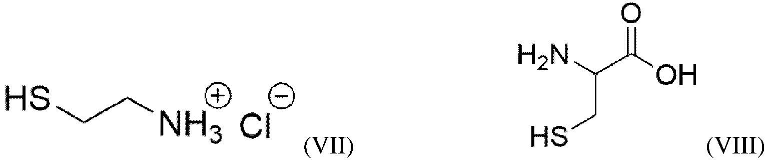

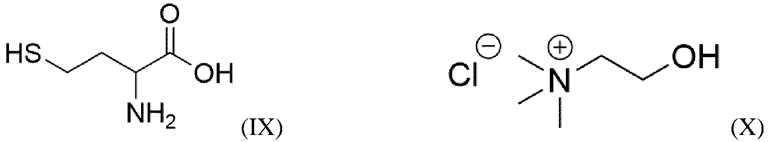

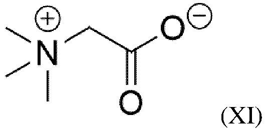

10. Procédé selon l'une quelconque des revendications précédentes, ***dans lequel*** le composé C1 et le composé C2 sont des composés issus au moins partiellement de sources renouvelables et non fossiles.

11. Procédé selon l'une quelconque des revendications précédentes, ***dans lequel*** le composé C1 et le composé C2 présentent une teneur en carbone d'origine renouvelable et non fossile comprise entre 5 % en poids et 100 % en poids selon la norme ASTM D6866-21, méthode B.

12. Procédé selon l'une quelconque des revendications précédentes, ***dans lequel*** le procédé comprend les étapes successives suivantes :
(1) ajout d'un polymère fonctionnalisé non anionique P3 à une suspension aqueuse de fibres cellulosiques ;
(2) formation d'une feuille de papier, de carton ou analogue ;
(3) séchage de ladite feuille de papier, de carton ou analogue.

13. Procédé selon l'une quelconque des revendications précédentes, ***dans lequel*** le procédé est mis en œuvre à l'aide d'énergie provenant d'une pompe à chaleur, d'un réseau de chaleur fatale, d'une source renouvelable, d'une pile à combustible, d'une batterie au lithium ou d'une centrale nucléaire.
